# EUROPEAN PATENT APPLICATION

(11) **EP 2 199 305 A1**
(43) Date of publication of application: **23.06.2010**
(21) Application number: 08075953.3
(22) Date of filing: 18.12.2008
(51) Int. Cl.: C07K 14/705, A61K 38/00

(54) **Peptides against autoantibodies associated with CRPS and use of these peptides**

(71) Applicant: Max-Delbrück-Centrum, 13125 Berlin (DE); Justus-Liebig-Universität Gießen, 35390 Giessen (DE)
(72) Inventor: Wallukat, Gerd Dr., 13129 Berlin (DE); Blaes, Franz Dr., 35447 Reiskirchen (DE)
(74) Representative: Boeckh, Tobias

(57) **Abstract**

The invention relates to nucleic add molecules encoding peptides which interact with autoantibodies associated with Complex Regional Pain Syndrome, to the peptides themselves, to a pharmaceutical composition comprising said nucleic add molecules and peptides, and to the use of said peptides - especially in apheresis and in vitro assays- for the treatment and the diagnosis of Complex Regional Pain Syndrome.

SEQUENCE LISTING

<110>  Max-Delbrück-Centrum für Molekulare Medizin

<120>  Peptides Against Autoantibodies associated with CRPS and Use of
       these Peptides

<130>  XII 662-08

<160>  15

<170>  PatentIn version 3.5

<210>  1
<211>  6
<212>  PRT
<213>  Artificial

<220>
<223>  Peptide capable of binding antibodies associated with Complex
       Regional Pain Syndrom

<400>  1

Val Arg Thr Val Glu Asp
1               5


<210>  2
<211>  6
<212>  PRT
<213>  Artificial

<220>
<223>  Peptide capable of binding antibodies associated with Complex
       Regional Pain Syndrome

<400>  2

Glu Asp Gly Glu Cys Tyr
1               5


<210>  3
<211>  10
<212>  PRT
<213>  Artificial

<220>
<223>  Peptide capable of binding antibodies associated with Complex
       Regional Pain Syndrome

<400>  3

Val Arg Thr Val Glu Asp Gly Glu Cys Tyr
1               5                   10


<210>  4
<211>  25
<212>  PRT
<213>  Artificial

<220>
<223>  Peptide capable of binding antibodies associated with Complex
       Regional Pain Syndrome

<400>  4

Val Arg Thr Val Glu Asp Gly Glu Cys Tyr Ile Gln Phe Phe Ser Asn
1               5                   10                  15

Ala Ala Val Thr Phe Gly Thr Ala Ile
            20                  25

<210>  5
<211>  27
<212>  PRT
<213>  Artificial

<220>
<223>  Peptide capable of binding antibodies associated with Complex
       Regional Pain Syndrome

<400>  5

His Trp Tyr Arg Ala Thr His Gln Glu Ala Ile Asn Cys Tyr Ala Asn
1               5                   10                  15

Glu Thr Cys Cys Asp Asp Phe Phe Thr Asn Gln
            20                  25

<210>  6
<211>  6
<212>  PRT
<213>  Artificial

<220>
<223>  Peptide capable of binding antibodies associated with Complex
       Regional Pain Syndrome

<400>  6

His Trp Tyr Arg Ala Thr
1               5

<210>  7
<211>  7
<212>  PRT
<213>  Artificial

<220>
<223>  Peptide capable of binding antibodies associated with Complex
       Regional Pain Syndrome

<400>  7

Ala Thr His Gln Glu Ala Ile
1               5

<210>  8
<211>  11
<212>  PRT
<213>  Artificial

<220>
<223>  Peptide capable of binding antibodies associated with Complex
       Regional Pain Syndrome

<400>  8

His Trp Tyr Arg Ala Thr His Gln Glu Ala Ile
1               5                   10

<210>  9
<211>  7
<212>  PRT
<213>  Artificial

<220>
<223>  Peptide of the beta2-adrenoceptor

<400>  9

Ala Ile Asn Cys Tyr Ala Asn
1               5

<210>  10
<211>  7
<212>  PRT
<213>  Artificial

<220>
<223>  Peptide of the beta2-adrenoceptor

<400>  10

Ala Asn Glu Thr Cys Cys Asp
1               5

<210>  11
<211>  6
<212>  PRT
<213>  Artificial

<220>
<223>  Peptide of the beta2-adrenoceptor

<400>  11

Asp Phe Phe Thr Asn Gln
1               5

<210>  12
<211>  6
<212>  PRT
<213>  Artificial

<220>
<223>  Peptide of the m2-receptor

<400>  12

Cys Tyr Ile Gln Phe Phe
1               5

<210>  13
<211>  6
<212>  PRT
<213>  Artificial

<220>
<223>  Peptide of the m2-receptor

<400>  13

Phe Phe Ser Asn Ala Ala
1               5


<210>  14
<211>  6
<212>  PRT
<213>  Artificial

<220>
<223>  Peptide of the m2-receptor

<400>  14

Ala Ala Val Thr Phe Gly
1               5


<210>  15
<211>  6
<212>  PRT
<213>  Artificial

<220>
<223>  Peptide of the m2-receptor

<400>  15

Val Thr Phe Gly Ala Ile
1               5

## Description

The invention relates to the use of a specific ligand for autoantibodies in the manufacture of a column for the treatment and/or diagnosis of Complex Regional Pain Syndrome.

Complex Regional Pain Syndrome surprisingly is often accompanied by the prevalence of antibodies directed against beta-2-adrenergic receptor and/or m2 muscarinic receptor. These antibodies can act as agonistic autoantibodies; these autoantibodies activate for example the beta-2-adrenergic receptor and/or the m2 muscarinic receptor or the beta-adrenergic system and/or the muscarinic system.

The invention relates more specifically to nudeic add molecules encoding peptides which interact with autoantibodies associated with Complex Regional Pain Syndrome, to the peptides themselves, to a pharmaceutical composition comprising said nudeic acid molecules and/or peptides, and to the use of said peptides - especially in apheresis or *in vitro* assays - for the treatment, follow-up treatment, aftercare, prophylaxes and/or diagnosis of Complex Regional Pain Syndrome.

Polycellular organisms, especially mammals, and more particularly humans, cope with their environment via various biochemical and/or immunological regulatory mechanisms. For example, an important biochemical regulatory mechanism is the one that is responsible for maintaining constant a specific internal temperature in the organism. Another important regulatory mechanism provides cellular and humoral substances, such as antibodies, enabling the organism to cope successfully with microorganisms, parasites, but also, tumors or other diseases. Above all, the function of antibodies is based on the capability of distinguishing between "foreign" and "self". That is, the organism itself produces antibodies which are designed in such a way that endogenous structures such as tissues or organs are not attacked, but bind to bacteria, parasites or other exogenic components, thus initiating or supporting immunological reactions.

During biochemical development of an antibody response in the organism, somatic hypermutations of antibody-producing B cells occur during T cell-dependent immune response. Such mutations selectively involve immunoglobulin genes, being essential to the formation of precursor cells of antibody-forming cells with high affinity. It should be noted in this context that such mutation is a normal and essentially non-pathogenic process. However, mutations may also give rise to the formation of highly affine autoantibodies, thus doing damage to the organism.

Autoantibodies are closely associated with the development of autoimmune diseases or causally responsible for such diseases. Antibody-mediated autoimmune diseases also include diseases such as rheumatism or lupus erythematosus. Numerous autoimmune diseases, being highly inconvenient to affected persons, relate to important biochemical cycles in the organism.

Complex Regional Pain Syndrome is a severe pain syndrome which is often associated with trophic disorders, like bone loss, impaired circulation or impaired temperature regulation.

Complex Regional Pain Syndrome and can be divided into two types based on the presence of nerve lesion following the injury.
Type I, also known as Reflex Sympathetic Dystrophy (RSD), Sudeck's atrophy, Reflex Neurovascular Dystrophy (RND) or Algoneurodystrophy, does normally not show demonstrable nerve lesions.
Type II, also known as Causalgia, has evidence of obvious nerve damage.
The cause of this syndrome is currently unknown. Precipitating factors include illness, injury and surgery, but there are also cases that have no documentable injury to the original site.

0.2% - 0.5% of all trauma patients develop Complex Regional Pain Syndrome subsequent to a limb trauma or a surgery. However, the syndrome can also occur spontaneously. Complex Regional Pain Syndrome affects between 5-10/100,000 citizens

Complex Regional Pain Syndrome is a progressive disease, which is often chronic and resistant to conventional therapy. There is no satisfying treatment of Complex Regional Pain Syndrome available so far. The conventional treatment is pain therapy, due to the administration of pain killers. The use of beta blocker in pain treatment is also described in the state of the art. It is obvious that these kinds of treatments are not ideal, because the effect does not last. Up to 50% of the patients show a chronic course of disease, in which case long-time pain and occupational therapy becomes necessary.
Another drawback is the fact that it often takes a long time until Complex Regional Pain Syndrome can be diagnosed. The deficiency in diagnosis is considered as a major problem. So far there is no simple and direct way to diagnose Complex Regional Pain Syndrome. Conventional diagnoses of Complex Regional Pain Syndrome results from the examination of a detailed symptom catalog. 3-Phase Bone Scans help to identify the disease in 60-79% of the patients. This is an expansive method which can only be performed in special medical practices. 60-79% success might be sufficient for therapy but it is an unsatisfying result for diagnosis, because up to 40% of the patients suffering from Complex Regional Pain Syndrome cannot be diagnosed. So far there is no laboratory assay for the diagnosis or any other test available, that allow distinguishing Complex Regional Pain Syndrome from other diseases. A lot of patients report seeing an average of five physicians before being accurately diagnosed. However, early and accurate diagnosis and appropriate treatment are key to recovery.

The object of the invention was therefore to provide compounds allowing easy, reliable and effective diagnosis and therapy of Complex Regional Pain Syndrome.

The invention solves the above technical problem by providing peptides with the following function:
All peptides of the invention are capable of binding antibodies associated with Complex Regional Pain Syndrome. A Group of peptides of the invention is not disclosed in the state of art; these peptides are claimed as product claims. The new peptides provided by the invention consist of an amino acid sequence X1-X2-X3-X4-X5-X6-X7-X8-X9-X10-X11-X12-X13 wherein

| | |
|---|---|
| X1 = | amino group, amide, acetyl group, biotin group, marker, spacer, linker, GKK, SGKK or deletion, |
| X2 = | H, A, V, E, L, D, I, N |
| X3 = | W, T, R, D, F, Y, S, C, K, G, E |
| X4 = | Y,H,T,G,S,C,R,K |
| X5 = | R, Q, V, E, K, G, N, D, I, L, A |
| X6 = | A, E, D, C, L, V, Y, S, T, N |
| X7 = | T, A, Y, D, G, S, C, L, V, K, R, Q |
| X8 = | I, H, E, G, L, V, D, N, K |
| X9 = | Q, C, E, N, S, T |
| X10= | E, Y, C, D, S, T, N, M |
| X11= | A, Y, L, V, S, T |
| X12= | I, L, V |
| X13= | amino group, amide, acetyl group, biotin group, marker, spacer, linker, GKK, SGKK or deletion, |

wherein the amino acid sequence is not VRTVED (SEQ ID NO 1), EDGECY (SEQ ID NO 2), or VRTVEDGECY (SEQ ID NO 3),.

Surprisingly all peptides consisting of an amino acid sequence X1-X2-X3-X4-X5-X6-X7-X8-X9-X10-X11-X12-X13 wherein

| | |
|---|---|
| X1 = | amino group, amide, acetyl group, biotin group, marker, spacer, linker, GKK, SGKK or deletion, |
| X2 = | H, A, V, E, L, D, I, N |
| X3 = | W, T, R, D, F, Y, S, C, K, G, E |
| X4 = | Y, H, T, G, S, C, R, K |
| X5 = | R, Q, V, E, K, G, N, D, I, L, A |
| X6 = | A, E, D, C, L, V, Y, S, T, N |
| X7 = | T, A, Y, D, G, S, C, L, V, K, R, Q |
| X8 = | I, H, E, G, L, V, D, N, K |
| X9 = | Q, C, E, N, S, T |
| X10= | E, Y, C, D, S, T, N, M |
| X11= | A, Y, L, V, S, T |
| X12= | I, L, V |
| X13= | amino group, amide, acetyl group, biotin group, marker, spacer, linker, GKK, SGKK or deletion, |

are capable of binding and neutralizing autoantibodies associated with Complex Regional Pain Syndrome.

It is preferred that the amino acid sequence or a part thereof is a part of the sequence VRTVEDGECYIQFFSNAAVTFGTAI (SEQ ID NO 4), and/or HWYRATHQEAINCYANETCCDDFFTNQ (SEQ ID NO 5). Especially preferred the peptides comprise the sequence HWYRAT (SEQ ID NO 6), ATHQEAI (SEQ ID NO 7), HWYRATHQEAI (SEQ ID NO 8), VRTVED, EDGECY and/or VRTVEDGECY.
It was not known to date that Complex Regional Pain Syndrome is a group of diseases which is associated with autoantibodies. In the controversial state of the art, an autoimmune etiology was discussed for Complex regional pain syndrome but at the same time other findings did not suggest the involvement of autoantibodies in the pathogenesis of Complex Regional Pain Syndrome. It was however utterly surprising, that ligands for autoantibodies may be applied in the therapy and diagnosis of Complex Regional Pain Syndrome.

According to the invention Complex Regional Pain Syndrome is a Reflex Sympathetic Dystrophy (RSD), a Causalgia, a Sudeck's atrophy, an algodystrophy, a neurodystrophic Syndrome, a dystrophic Pseudoarthritis, Reflex Neurovascular Dystrophy (RND), an Algoneurodystrophy and/or a transiente Osteoporose.

In terms of the invention, Complex Regional Pain Syndrome encompasses every disease associated with pain. Each disease that shows one or more of the following symptoms is Complex Regional Pain Syndrome according to the invention:
Continuous burning pain in the distal part of the affected extremity,
Pain is disproportionate in intensity to the inciting event and usually increases when the extremity is in a dependent position,
Stimulus-evoked pains include mechanical and thermal allodynia and/or hyperalgesia, and deep somatic allodynia (pain due to touching the joints and/or movement of joints),
Sensory abnormalities are most pronounced distally, and/or have no consistent spatial relationship to individual nerve territories and/or to the site of the inciting lesion,
Swelling of the distal extremity especially in the acute phase,
Hyper- or hypohidrosis,
Vasodilatation or vasoconstriction,
Impaired circulation,
Bone loss,
Changes in skin temperature,
Abnormal nail growth,
Increased or decreased hair growth,
Fibrosis,
Thin, glossy skin,
Osteoporosis,
Weakness,
Coordination deficits,
Tremor,
Dystonia,

Neglect-like symptoms and/or symptoms of disturbed body perception of the affected extremity; preferred:
Preceding noxious event with nerve lesion,
Preceding noxious event without nerve lesion,
Spontaneous pain or hyperalgesia/hyperesthesia not limited to a single nerve territory and disproportionate to the inducing event,
Edema, skin blood flow (temperature) or sudomotor abnormalities,
Motor symptoms or trophic changes are present on the affected limb, in particular at distal site.

Also preferred is the peptide in a linear and branched as well as cyclic form, wherein the peptide ring closure is effected through disulfide bridging when two cysteines are present, or through amide cyclization, which is optionally effected through side chains, through the C and N termini or through a combination of these three possibilities.
Cyclic peptides are especially preferred due to their resistance to digestion. Therefore cyclic peptides can be used advantageous for example in the production of a pharmaceutical composition.

In another aspect, the invention provides an isolated nucleic add molecule - and a peptide encoded by same - selected from the group comprising:
a) a nudeic acid molecule comprising a nucleotide sequence which encodes at least one peptide selected from the group consisting of peptides according to claim 1, preferred HWYRAT, ATHQEAI and/or HWYRATHQEAI;
b) a nudeic acid molecule which is complementary to a nucleotide sequence in accordance with a);
c) a nudeic add molecule which undergoes hybridization with a nucleotide sequence according to a) or b) under stringent conditions;
d) a nucleic add molecule comprising a nucleotide sequence having sufficient homology to be functionally analogous to a nucleotide sequence according to a), b) or c);
e) a nudeic acid molecule which, as a consequence of the genetic code, is degenerated into a nucleotide sequence according to a) through d); and
f) a nudeic add molecule according to a nucleotide sequence of a) through e) which is modified by deletions, additions, substitutions, translocations, inversions and/or insertions and functionally analogous to a nucleotide sequence according to a) through e)

Accordingly, the invention involves the surprising teaching that the nudeic acid molecules according to the invention, as well as the peptides encoded by same, can be used for diagnosis and therapy of Complex Regional Pain Syndrome. More specifically, the biological structures, preferably peptides, encoded by said nudeic add molecules can be used in various forms in the prophylaxis, diagnosis, therapy, follow-up and/or aftercare of Complex Regional Pain Syndrome.

Surprisingly autoantibodies associated with Complex Regional Pain Syndrome are directed against receptors located on neurons, preferred neurons of the autonomic nervous system.
In a preferred embodiment of the invention the peptides are derived from the β2 adrenergic receptor and/or the m2 muscarinic receptor.
It was especially surprising that sequences derived from the β2 adrenergic receptor can be used in the diagnosis and therapy of Complex Regional Pain Syndrome. Antibodies directed against sequences of the β2 adrenergic receptor are known to play a role in the clinical picture of Chagas disease. But it was very surprising that there are also antibodies against the β2 adrenergic receptor in patients with Complex Regional Pain Syndrome. Due to the wide differences between Chagas disease and Complex Regional Pain Syndrome it could have not been expected that the autoantibodies of both diseases are directed against the same or similar structures.
All patients suffering from Complex regional pain syndrome show at least one type of the described autoantibodies, whereas most patients have both, antibodies against the β2 adrenergic receptor and antibodies against the m2 muscarinic receptor.

In a preferred embodiment of the invention the nucleic add molecule which has sufficient homology to be functionally analogous to the nucleic acid sequence in accordance with a), b) and/or c) has at least 40% homology. In the meaning of the invention the term "to be functionally analogous to the above-mentioned nucleotide sequences or to the sequences hybridizing with said nucleotide sequences" means that the homologues exhibit a behavior in Complex Regional Pain Syndrome, such that reliable and effective use thereof is possible in diagnosis and/or therapy of such diseases or pathogenic conditions associated with said diseases. Functionally analogous sequences in the meaning of the invention are all those sequences which can be identified as equally effective by a person skilled in the art, using routine tests.

In another advantageous embodiment of the invention the nucleic acid molecule has at least 60%, preferably 70%, more preferably 80%, especially preferably 90% homology to the nudeic acid molecule in accordance with d).

In another preferred embodiment of the invention the nucleic acid molecule is a genomic DNA, a cDNA and/or a RNA.

Also preferred is an isolated nucleic acid molecule selected from the group comprising:
a) a nudeic acid molecule comprising a nucleotide sequence which encodes at least one peptide selected from the group consisting of peptides according to claim 1 and/or 8;
b) a nucleic acid molecule which is complementary to a nucleotide sequence in accordance with a);
c) a nudeic add molecule which undergoes hybridization with a nucleotide sequence according to a) or b) under stringent conditions;
d) a nudeic add molecule comprising a nucleotide sequence having sufficient homology to be functionally analogous to a nucleotide sequence according to a), b) or c);
e) a nudeic add molecule which, as a consequence of the genetic code, is degenerated into a nucleotide sequence according to a) through d); and
f) a nudeic add molecule according to a nucleotide sequence of a) through e) which is modified by deletions, additions, substitutions, translocations, inversions and/or insertions and functionally analogous to a nucleotide sequence according to a) through e)
for use in prophylaxis, diagnosis, therapy, follow-up and/or aftercare of Complex Regional Pain Syndrome.

It was very surprising, that these nudeic adds could be used successfully in prophylaxis, diagnosis, therapy, follow-up and/or aftercare of Complex Regional Pain Syndrome. Preferred is the use of nudeic acids encoding the peptides with sequences HWYRAT, ATHQEAI, HWYRATHQEAI, VRTVED, EDGECY or VRTVEDGECY.
Also preferred is the use of these nudeic add sequences in the manufacture of a medicament for the treatment of Complex Regional Pain Syndrome.

In another preferred embodiment the nucleic acid sequences are use to manufacture an agent for the diagnosis of Complex Regional Pain Syndrome.

The invention also relates to a vector comprising a nudeic add molecule according to the invention and, in addition, to a host cell comprising said vector, said nudeic add molecule and/or said peptide.

In a preferred fashion the invention also relates to a peptide encoded by a nudeic acid molecule according to the invention and by the preferred functionally analogous nucleic acid molecules. Surprisingly, the peptide according to the invention can be used in the diagnosis and/or therapy of diseases associated with Complex Regional Pain Syndrome. More specifically, the peptides of the invention are bound by autoantibodies in patients suffering from Complex Regional Pain Syndrome.

That is, the invention relates to all peptides encoded by the nudeic acid molecules according to the invention, preferably those having at least 60%, preferably 70%, more preferably 80%, especially preferably 90% homology to the nucleic add molecule in accordance with d). Obviously, said functionally analogous nudeic acid molecules can be modified by deletion, addition, substitution, translocation, inversion and/or insertion in such a way that the peptides encoded by same interact with autoantibodies associated with Complex Regional Pain Syndrome. By virtue of the teaching according to the invention, a person skilled in the art will be capable of generating other equivalent peptides functionally analogous to peptides having the sequence of claim 1 and 8, preferred HWYRAT, ATHQEAI, HWYRATHQEAI, VRTVED, EDGECY and/or VRTVEDGECY, respectively. Of course, it is also possible to use peptides comprising the above-mentioned structures. Obviously, however, the peptides (as a substance, molecule or a mixture of substances) according to the invention would not be selected in a way so as to completely represent the naturally occurring human beta-2-adrenergic receptor and/or m2 muscarinic receptor. With reference to the methods of the invention and the uses according to the invention, it can be advantageous to use the human beta-2-adrenergic receptor and/or m2 muscarinic receptor or parts thereof as specific ligands for removing significant portions of the immunoglobulin from plasma taken from a patient, suffering from Complex Regional Pain Syndrome. This means that the substance claims refer to specific parts of the receptor (the peptides) and the use or method claims refer to the use of the whole receptor for the treatment of Complex Regional Pain Syndrome. A person skilled in the art knows the definition of a ligand according to the invention. Ligands or specific ligands in the context of the invention are means which interact specifically with immunoglobulins, preferred autoantibodies, more preferred autoantibodies directed against the beta-2-adrenergic receptor and/or the m2 muscarinic receptor, most preferred against the second extracellular loop of the beta-2-adrenergic receptor and/or the m2 muscarinic receptor. In another preferred embodiment of the invention, the ligands or specific ligands consist of the peptides of the invention. The autoantibodies associated with Complex Regional Pain Syndrome can be bound very well with the peptides of the invention. If a less specific binding is necessary, the invention discloses the technical teaching for extracting the antibodies or autoantibodies from the plasma or blood of a human (for example by using an unspecific column) and for consequently reinfusing the needed antibodies to the patient, needed antibodies being such which are important for the immune system of a patient. So far there are no peptides known in the state of art that may be used for generating antibodies which can be used for the treatment or the diagnosis of Complex Regional Pain Syndrome.

In another preferred embodiment, the invention relates to the use of a specific ligand for human immunoglobulin in the manufacture of a column having said ligand coupled thereto for the treatment of a patient suffering from Complex Regional Pain Syndrome, said treatment comprising passing plasma of the patient over the column under conditions which effect the binding of said specific ligand to immunoglobulin in the patient's plasma, thereby removing a significant portion of the immunoglobulin from the patient's plasma and reinfusing the plasma to the patient. In the context of the invention, the term "significant portion" is directed to an amount of autoantibodies whose removal from the plasma leads to an improvement of the patient's condition or to the non-appearance of a further aggravation. This improvement of the patient's condition relates to at least one of the aspects of Complex Regional Pain Syndrome, known to a person skilled in the art In a preferred embodiment of the invention, the specific ligand is the beta-2-adrenergic receptor and/or the m2 muscarinic receptor or the peptides according to the invention. These aspects of the invention are based on the conclusion made by the inventors that Complex Regional Pain Syndrome is caused by autoantibodies, especially by agonistic autoantibodies.

The invention also encompasses the use of a specific ligand in the manufacture of a column for extracorporal removal of autoantibodies directed against Complex Regional Pain Syndrome structures by removing immonglobulins of any or all classes and subclasses, for the treatment of Complex Regional Pain Syndrome. Such removal can be accomplished by using any specific ligands for human immunoglobulin coupled to the IA column. Such ligands include polyclonal and monoclonal anti-human immunoglobulin antibodies, fragments of such antibodies (FAB₁, FAB₂), recombinant antibodies or proteins, synthesized peptides, Protein A and Protein G. The term "Complex Regional Pain Syndrome structures" is known to a person skilled in the art and refers to structures associated with this disease. The term "Complex Regional Pain Syndrome structure" therefore refers to any structure in the human body which is modified by Complex Regional Pain Syndrome or whose modification causes Complex Regional Pain Syndrome. A "Complex Regional Pain Syndrome structure" is a binding site of said autoantibody, especially of agonistic autoantibodies.

Another aspect of the invention is therefore also the essentially unspecific removal of immunoglobulins from plasma taken from a patient suffering from Complex Regional Pain Syndrome and reinfusing said plasma to the patient. The reinfused plasma may also contain those immunoglobulins which induce a positive effect in the context of the patent's immune system.

The invention also encompasses the use of more specific ligands in the manufacture of a column for extracorporal removal of autoantibodies which are directed against the second extracellular loop of the beta-2-adrenergic receptor and/or the m2 muscarinic receptor. This means that the removal of all antibodies or the removal of all autoantibodies or the removal of all autoantibodies directed against the second extracellular loop of the beta-2-adrenergic receptor and/or the m2 muscarinic receptor is part of the invention.

In another preferred embodiment, the peptides according to the invention relate to selected regions of the second extracellular loop of the beta-2-adrenergic receptor and/or the m2 muscarinic receptor. However, the advantageous sequences X1-X2-X3-X4-X5-X6-X7-X8-X9 -X10-X11-X12-X13 wherein

| | |
|---|---|
| X1 = | amino group, amide, acetyl group, biotin group, marker, spacer, linker, GKK, SGKK or deletion, |
| X2 = | H, A, V, E, L, D, I, N |
| X3 = | W, T, R, D, F, Y, S, C, K, G, E |
| X4 = | Y, H, T, G, S, C, R, K |
| X5 = | R, Q, V, E, K, G, N, D, I, L, A |
| X6 = | A, E, D, C, L, V, Y, S, T, N |
| X7 = | T, A, Y, D, G, S, C, L, V, K, R, Q |
| X8 = | I, H, E, G, L, V, D, N, K |
| X9 = | Q, C, E, N, S, T |
| X10= | E, Y, C, D, S, T, N, M |
| X11= | A, Y, L, V, S, T |
| X12= | I, L, V |
| X13= | amino group, amide, acetyl group, biotin group, marker, spacer, linker, GKK, SGKK or deletion |

may also comprise further amino adds naturally flanking the above-mentioned sequences in the second extracellular loop of the beta-2-adrenergic receptor and/or the second extracellular loop of the m2 muscarinic receptor, in which case the size of the flanking regions should not be selected such that the receptors themselves would be selected. Examples of such sequences - expanded by flanking or other artificial regions - are HWYRATHQE-Seq - a peptide of the invention with a sequence of the extracellular loop flanking N-terminal region. In this case the flanking sequence is representing a linker or spacer of a part thereof.

Consequently, the peptides in the meaning of the invention can be designed in such a way and may include such a number of additional amino acids, spacers or other structures that they are suitable for interaction with the antibodies, and preferably in such a way that they represent an epitope for the latter.

Accordingly, the peptides according to the invention are not restricted to the sequences HWYRAT, ATHQEAI, HWYRATHQEAI, VRTVED, EDGECY and/or VRTVEDGECY, but rather, the peptides are preferably antibody epitopes essentially including the above-mentioned or functionally analogous sequences, so that they represent and characterize the epitope in such a way that the antibodies would undergo specific interaction with them. Therefore, under particular conditions the terms "epitope" and "peptide" may also be used synonymously.

Furthermore, it is possible to replace single amino acids or groups of amino acids without adversely affecting the activity of the peptides with respect to accomplishing the object of the present invention. For replacement of such amino acids, reference is made to appropriate standard textbooks of biochemistry and genetics. Various ways of preparing peptides have been disclosed in the prior art. Peptides designed starting from the peptides of the invention using such methods are included in the teaching according to the invention. For example, one way of generating functionally analogous peptides has been described in PNAS USA 1998, Oct. 13, 95, 12179-84; WO 99/6293 and/or WO 02/38592; and the above teachings are hereby incorporated in the disclosure of the invention.

A second way of generating peptide sequences starting from the peptides of invention is the subtitutional analysis (Mol. Diversity 2004, 8, 281-290; J. of Immunol. Methods 2002, 267, 37-51 The substitutional analysis is carried out by systematical replacement of each amino acid by all amino acids/building blocks of your interest. The activity of these peptides/constructs shows the ability of those substitutions. The resulting sequences could have improved properties i.e. higher stability and/or lower hydrophicity).

Another method to generate peptide structure anologues is the synthesis of retro-inverso peptides. Using this method the L-amino acids of the peptides of invention were substituted by D-amino acids with simultaneously inversion of the direction of the peptide bounds. This method is published e.g. in Biochemistry 2001, Apr. 19, 40, 5720-27; Biopolymers (Peptide Science) 2005, Feb. 23, 80, 67-84. The generated peptides are structurally analogue to the peptides of the invention. But due to the use of D-amino acids, these peptides are stable against proteolytic degradation. One example of such peptide sequence is the sequence: naycniG. This sequence is the retro-inverso sequence of a peptide of the invention, in which several amino acids were replaced by other amino adds with similar properties. The small letters indicate D-amino acids. Glycine (G) doesn't exist in two different D- or L-structures.

That is, all peptides, peptide fragments or structures comprising peptides generated using the methods mentioned above - starting from the peptides of the invention - are peptides according to the invention, provided they accomplish the object of the invention and, in particular, interact with the pathogenic autoantibodies. For example, these autoantibodies - via dimerization - can be agonistic autoantibodies activating receptors. Some preferred receptors will be described below.

For example, the peptide sequences can be a natural component of the second extracellular loop of the human beta-2-adrenergic receptor and/or a natural component of the second extracellular loop of the human m2 muscarinic receptor. Surprisingly, the autoantibodies specifically interact with the second extracellular loop or with the loop of the respective beta-2-adrenergic and/or the m2 muscarinic receptor. Accordingly, the invention also relates to the surprising teaching that the major epitope of the autoantibodies associated with Complex Regional Pain Syndrome are peptides having the amino acid sequences of claim 1 or 8, preferred HWYRAT, ATHQEAI, HWYRATHQEAI, VRTVED, EDGECY and/or VRTVEDGECY. Preferably, this sequence is entirely identical on beta-2-adrenergic receptor and/or the m2 muscarinic receptor. Accordingly, the autoantibodies can be antibodies acting as agonistic autoantibodies, i.e., these autoantibodies are capable of activating the corresponding receptors by binding thereto. As a result of such receptor activation, various diseases or pathogenic changes can be induced and/or accompanied. For example, it is possible that activation of receptors modulates the production of messenger molecules. However, the autoantibodies may also damage receptors of cells which are responsible for essentially normal blood flow in limbs. In contrast to the effect of agonistic autoantibodies, the function of attacking and damaging the corresponding target by chronic stressing - usually observed with antibodies - is the predominant one in non-agonistic autoantibodies. However, this does not exclude activation or deactivation of the receptors.

In a preferred embodiment of the invention the peptide additionally comprises amino groups, amides, acetyl groups, biotin groups, markers, spacers and/or linkers.

By virtue of such structures, the peptide can be used with advantage in various fields of diagnosis and therapy of autoimmune diseases associated with pain. Various ways of modifying peptides for various applications are well-known to those skilled in the art. If, for example, the peptides are envisaged to be administered as a drug, the structure of the peptides has to be changed in a specific fashion, as is well-known to those skilled in the art. However, the peptides may also be bound to the supporting material of an affinity column in order to be used in the purification of body fluids, especially blood; binding of the peptides to a matrix requires specific structural modifications of the peptides according to the invention, and such modifications are also well-known to those skilled in the art or can be determined using routine tests.

As is well-known to those skilled in the art, some amino acids have analogous physicochemical properties so that these amino acids advantageously can be replaced by each other. For example, these include the group of nonpolar (hydrophobic) amino acids (a) glycine, alanine, valine, leucine and/or isoleucine; or the hydroxy amino acids (b) serine, threonine and/or tyrosine, the amides of amino dicarboxylic adds (c) asparagine and glutamine, the amino dicarboxylic acids (d) aspartic acid and glutamic acid; the basic amino adds (e) lysine, arginine and/or ornithine as well as the group of aromatic amino acids (f) phenylalanine, tyrosine and/or tryptophan. Another aspect is the replacement of amino acids by structural similar amino acids. For example this is the case in the group with a β-fundctional group (g) cysteine, methionine, serine, α- aminobutyric acid and selenocysteine as well as the tum-inducing group (h) praline, 1-amino-2-carboxy cyclohexane, pipecolic acid and an ortho-aminobenzoic acid. Amino acids within one and the same group (a-h) can be replaced with one another. In all cases, peptide sequences will have a sufficient homology to be an analogous to an amino add sequence of the peptides of the invention. Furthermore, the amino acids can be replaced by modified amino adds or specific enantiomers. Further modifications are possible in accordance with the teaching of WO 99/62933 or WO 02/38592. For example it is possible to replace several amino acids of the peptides of the invention by other amino acids with similar physiochemical or structural properties. It is obviously known to a person skilled in the art that it is possible to provide functionally analogous means for the peptides and nudeic adds of the invention. In the meaning of the invention, "functionally analogous means" are means which are functionally equivalent or equivalent to the peptides and nudeic acids of the invention. In order to check whether a sequence is functionally equivalent (= equivalent) to the peptides or nudeic acids of the invention, one must check whether the functionally analogous/equivalent sequence of the peptide or of the nudeic acid produces essentially the same function in essentially the same way with essentially the same outcome. That is, the outcome produced with the peptides or nudeic acids of the invention may also be produced with the functionally analogous/equivalent means. A person skilled in the art can therefore easily and quickly verify whether a functionally analogous/equivalent sequence provided by them is covered by the invention. The functionally analogous sequence is covered by the invention if it may be used for solving the problem of the invention and if the solving of the problem of the invention can be carried out in essentially the same way as is claimed by the invention. While the invention has been described in terms of preferred embodiments, the skilled artisan will appreciate that various modifications, substitutions, omissions and changes may be made without departing from the spirit thereof. Accordingly, it is intended that the scope of the present invention be limited solely by the scope of the claims of the invention, including equivalents thereof. Therefore, as will be apparent to those skilled in the art to which the invention is addressed, the present invention may be embodied in forms other than those specifically disclosed without departing from the spirit or essential characteristics of the invention. The particular embodiments of the present invention as described are therefore to be considered in all respects as illustrative and not restrictive. The scope of the present invention is as set forth in the appended claims rather than being limited to the examples contained in the foregoing description.

It should be understood that various alternatives to the embodiments of the invention described herein may be employed in practicing the invention. It is intended that the claims of the invention define the scope of the invention and that the method and apparatus within the scope of these claims and their equivalents be covered thereby.

Those skilled in the art will therefore understand all those molecules as equivalent which he may apply without this having an effect on the solution of the problem of the invention. They know that the claimed sequences must not be modified by changes, substitutions or deletions in a way that they cannot be used for solving the problem of the invention.

In another preferred embodiment the peptide comprises a linker and/or a spacer selected from the group comprising α-aminocarboxylic adds as well as homo- and heterooligomers thereof, α,ω-aminocarboxylic acids and branched homo- or heterooligomers thereof, other aliphatic and/or aromatic amino acids, as well as linear and branched homo- or heterooligomers (peptides); amino-oligoalkoxyalkylamines; maleinimidocarboxylic acid derivatives; oligomers of alkylamines; 4-alkylphenyl derivatives; 4-oligoalkoxyphenyl or 4-oligoalkoxyphenoxy derivatives; 4-oligoalkylmercaptophenyl or 4-oligoalkylmercaptophenoxy derivatives; 4-oligoalkylaminophenyl or 4-oligoalkylaminophenoxy derivatives; (oligoalkylbenzyl)phenyl or 4-(oligoalkylbenzyl)phenoxy derivatives, as well as 4-(oligoalkoxybenzyl)phenyl or 4-(oligoalkoxybenzyl)phenoxy derivatives; trityl derivatives; benzyloxyaryl or benzyloxyalkyl derivatives; xanthen-3-yloxyalkyl derivatives; (4-alkylphengyl) or ω-(4-alkylphenoxy)alkanoic acid derivatives; oligoalkylphenoxyalkyl or oligoalkoxyphenoxyalkyl derivatives; carbamate derivatives; amines; trialkylsilyl or dialkylalkoxysilyl derivatives; aliphatic or aromatic mono- or oligomercapto derivatives; alkyl or aryl derivatives and/or combinations thereof; other possible structures have been described in EP 1 214 350 which hereby is incorporated in the disclosure of the invention. The following sequence is an example: GLN-Cys(Maleinimidopropionic acid)-TYAN. In this sequence a peptide of the invention several amino acids were replaced by other with similar properties as described above. Additionally, the SH group of the cysteine reacts with the double bound of the maleinimide group to form a covalent bound to a linker molecule. This linkage is could couple to another molecule or a solid phase.

Also claimed according to the invention are solid phases for affinity chromatography or solid phase extraction constisting of organic, inorganic, synthetic polymers or of mixed polymers, preferably cross-linked agarose, cellulose, silica gel, polyamide and polyvinyl alcohols, which are optionally chemically activated, with peptides according to the invention immobilized on the surface of the solid phase.

In the solid phase according to the invention, the peptides are bound to the solid support phase preferably covalently or by adsorption. In another preferred embodiment of the solid phases according to the invention, the peptides are distanced from the support surface by linkers/spacers.

The invention relates to a method for the adsorption of immunoglobulins to a solid phase, wherein the peptides according to the invention are bound to a solid phase usual in affinity chromatography or to mobile solid phases, and the immunoglobulin-containing samples to be adsorbed are contacted, in particular, with the solid phases according to the invention.

Preferably, the method for the adsorption of immunoglobulins is performed on a solid phase, wherein the peptides according to the invention are bound to a solid phase usual in affinity chromatography or to mobile solid phases, and the immunoglobulin-containing samples to be adsorbed are anticoagulant-treated human blood plasma or human whole blood and are contacted with the solid phases according to the invention.

The method according to the invention is suitable, in particular, for the adsorption of immunoglobulins from immunoglobulin-containing samples which contain auto-antibodies related to autoimmune diseases, especially rheumatoid diseases, multiple sclerosis, myasthenia gravis and other auto-antibody-mediated diseases.

The method according to the invention is advantageously performed with a device according to the invention for removing immunoglobulins from immunoglobulin-containing samples on solid phases, wherein the device contains a solid phase according to the invention, and means for the entry of immunoglobulin-containing samples are provided.

The invention also relates to the use of the peptides according to the invention, the solid phases according to the invention and the devices according to the invention for removing immunoglobulins from immunoglobulin-containing samples.

According to another particularly preferred embodiment of the invention, the peptide is selected from the group comprising:
a) a peptide essentially containing the amino acid sequence according to claim 1 or 8, preferred HWYRAT, ATHQEAI, HWYRATHQEAI, VRTVED, EDGECY, and/or VRTVEDGECY;
b) a peptide comprising an amino add sequence having sufficient homology to be functionally analogous to an amino acid sequence in accordance with a);
c) a peptide according to an amino acid sequence a) or b) which is modified by deletions, additions, substitutions, translocations, inversions and/or insertions and functionally analogous to an amino acid sequence in accordance with a) or b).
d) a peptide according to amino acid sequence a), b) or c) which is modified by branch or extension with the same or another peptide according to amino acid sequence a), b) or c) to form a homooligomeric or heterooligomeric peptide. The coupling could result by a direct bond to amino acids of the sequences or by an indirect bond to amino acids of the sequences using a linker or spacer.

In a distinctive embodiment of the invention the peptide which has sufficient homology to be functionally analogous to one of the following amino acids of claim 1 or 8, preferred HWYRAT, ATHQEAI, HWYRATHQEAI, VRTVED, EDGECY, and/or VRTVEDGECY. In another preferred embodiment said amino acid sequences have at least 40% homology to one of the following amino acid sequences of claim 1, preferred HWYRAT, ATHQEAI, HWYRATHQEAI, VRTVED, EDGECY, and/or VRTVEDGECY.
In another preferred embodiment said amino acid sequences have at least 60%, preferably 70%, more preferably 80%, especially preferably 90% homology to one of the following amino acid sequences of claim 1, preferred HWYRAT, ATHQEAI, HWYRATHQEAI, VRTVED, EDGECY, and/or VRTVEDGECY.

In an especially preferred embodiment of the invention the peptide essentially consists of the amino acid sequence of claim 1 or 8, preferred HWYRAT, ATHQEAI, HWYRATHQEAI, VRTVED, EDGECY, and/or VRTVEDGECY. More specifically, the peptide consists of modifications of said sequences, which are obtained according to WO 99/62933 and WO 02/38592, and it goes without saying that these peptides bind autoantibodies in the meaning of the invention.

In another preferred embodiment of the invention the peptide is used or employed as a therapeutic active substance. In the meaning of the invention, use as a therapeutic active substance means use of the peptide in the entire field of medicine.

In another particularly preferred embodiment of the invention the peptide is bound by specific antibodies of patients with Complex Regional Pain Syndrome, especially by autoantibodies. At a defined quantity ratio of peptide and autoantibody, which is well-known to those skilled in the art, autoantibody-peptide complexes will form which, for example, undergo precipitation or exhibit specific reaction behavior in a way so as to allow elimination or reduction of the autoantibodies.

In another preferred embodiment of the invention the peptide, in particular, is immobilized. In the meaning of the invention, immobilization is understood to involve various methods and techniques to fix the peptides on specific carriers, e.g. according to WO 99/56126 or WO 02/26292. For example, immobilization can serve to stabilize the peptides so that their activity would not be reduced or adversely modified by biological, chemical or physical exposure, especially during storage or in single-batch use. Immobilization of the peptides allows repeated use under technical or clinical routine conditions; furthermore, a sample - preferably blood components - can be reacted with at least one of the peptides according to the invention in a continuous fashion. In particular, this can be achieved by means of various immobilization techniques, with binding of the peptides to other peptides or molecules or to a carrier proceeding in such a way that the three-dimensional structure - particularly in the active center mediating the interaction with the autoantibodies - of the corresponding molecules, especially of said peptides, would not be changed. Advantageously, there is no loss in specificity to the autoantibodies of the patient as a result of such immobilization. In the meaning of the invention, three basic methods can be used for immobilization:
(i) Crosslinking: in crosslinking, the peptides are fixed to one another without adversely affecting their activity. Advantageously, they are no longer soluble as a result of such crosslinking.
(ii) Binding to a carrier: binding to a carrier proceeds via adsorption, ionic binding or covalent binding, for example. Such binding may also take place inside microbial cells or liposomes or other membranous, dosed or open structures. Advantageously, the peptides are not adversely affected by such fixing. For example, multiple or continuous use of carrier-bound peptides is possible with advantage in clinics in diagnosis or therapy.
(iii) Inclusion: inclusion in the meaning of the invention especially is inclusion in a semipermeable membrane in the form of gels, fibrils or fibers. Advantageously, encapsulated peptides are separated from the surrounding sample solution by a semipermeable membrane in such a way that interaction with the autoantibodies or fragments thereof still is possible.

Various methods are available for immobilization, such as adsorption on an inert or electrically charged inorganic or organic carrier. For example, such carriers can be porous gels, aluminum oxide, bentonite, agarose, starch, nylon or polyacrylamide. Immobilization proceeds via physical binding forces, frequently involving hydrophobic interactions and ionic binding. Advantageously, such methods are easy to handle, having little influence on the conformation of the peptides. Advantageously, binding can be improved as a result of electrostatic binding forces between the charged groups of the peptides and the carrier, e.g. by using ion exchangers such as Sephadex.

Another method is covalent binding to carrier materials. In addition, the carriers may have reactive groups forming homopolar bonds with amino acid side chains. Suitable functional side chain groups in peptides are carboxy, hydroxy and hydrosulfide groups and especially the terminal amino groups of lysines. Aromatic groups offer the possibility of diazo coupling. The surface of microscopic porous glass particles can be activated by treatment with silanes and subsequently coated with peptides. For example, hydroxy groups of natural polymers can be activated with bromocyanogen and subsequently coupled with peptides. Advantageously, a large number of peptides can undergo direct covalent binding with polyacrylamide resins. Inclusion in three-dimensional networks involves inclusion of the peptides in ionotropic gels or other structures well-known to those skilled in the art. More specifically, the pores of the matrix are such in nature that the peptides are retained, allowing interaction with the target molecules. In crosslinking, the peptides are converted into polymer aggregates by crosslinking with bifunctional agents. Such structures are gelatinous, easily deformable and, in particular, suitable for use in various reactors. By adding other inactive components such as gelatin in crosslinking, advantageous improvement of mechanical and binding properties is possible. In microencapsulation, the reaction volume of the peptides is restricted by means of membranes. For example, microencapsulation can be carried out in the form of an interfacial polymerization. Owing to the immobilization during microencapsulation, the peptides are made insoluble and thus reusable. In the meaning of the invention, immobilized peptides are all those peptides being in a condition that allows reuse thereof. Restricting the mobility and solubility of the peptides by chemical, biological or physical means advantageously results in lower process cost, particularly when eliminating autoantibodies from blood components.

In another preferred embodiment of the invention the peptide is bound to a solid phase. Binding the peptide to the solid phase may proceed via a spacer. All those chemical compounds can be used as spacers which have the structural and functional preconditions suitable for the function of a spacer, provided they do not modify the binding behavior in such a way that binding of the autoantibody with the peptide would be adversely affected.

The invention also relates to recognition molecules directed against the agonistic autoantibodies which interact with the peptides of the invention. These agonistic autoantibodies are directed against the beta-2 receptor and/or the m2 muscarinic receptor. The interaction or reaction or binding of the autoantibody is a specific antigen-antibody-interaction. In a preferred embodiement the recognition molecules are antibodies, antisense constructs and/or chelating agents and/or aptamers or Spiegelmers. The recognition molecules according to the invention can be antibodies directed against the autoantibodies inducing e.g. Complex Regional Pain Syndrome; it is also possible that the recognition molecule is a peptidomimetic. To understand what a Spiegelmer is, it is important to first explain the concept of aptamers. An aptamer is a nudeic add structure that can bind to a target molecule conceptually similar to an antibody that recognizes an antigen (Ellington & Szostak, 1990). Based on the SELEX (Systematic Evolution of Ligands by EXponential enrichment) process (Tuerk & Gold, 1990), aptamers can be identified from huge combinatorial nudeic acids libraries of up to > 10¹⁵ different sequences. The sequences in the library are comprised of a central randomized region flanked by fixed sequences that permit amplification by polymerase chain reactions (PCR). The library size depends on the length of the random portion of the molecule, creating a diverse universe of molecules ready for screening. The aptamers capable of binding to a target are then selected by incubating the libraries with the immobilized target, washing extensively, then eluting the bound aptamer. After amplification by PCR, these binding molecules are reselected, eluted and amplified. By repeating this procedure multiple times, and eluting bound aptamers under progressively more stringent conditions, the molecules with greatest affinity and specificity are selected.

Aptamers have binding characteristics similar to peptides or antibodies, with affinities in the low nanomolar to the picomolar range. However, there are several drawbacks to aptamers as useful therapeutic products. As relatively small molecules, aptamers demonstrate circulating half-lives in vivo in the order of minutes. This situation can be addressed by attaching large inert molecules to aptamers (e.g. polyethylene glycol) to reduce their elimination via the kidney and increase their presence in the circulation. Finally, aptamers, as natural nudeic acid polymers, are prone to rapid degradation by nucleases that are present in all tissues in the body. To overcome this latter problem, methods of creating nuclease-resistant molecules that retained the binding capabilities of aptamers needed to be created. The answer to this latter problem was solved by the discovery and development of Spiegelmers (Klussmann et al., 1996; Vater & Klussmann, 2003). These molecules, which are biostable aptamers, have all of the diversity characteristics of aptamers but possess a structure that prevents enzymatic degradation. While aptamers are created from the natural D-nucleotides, which are recognized by the nudeic add degrading enzymes, a molecule synthesized as the mirror image L-oligonudeotide will not be degraded by any nuclease since there are no such enzymes in the body capable of interacting with these unnatural molecules. As such, the amplification methods used to create large aptamer libraries cannot be employed to synthesize the L-nucleic acid-containing molecules. However, in the state of the art there are known methods used to form high affinity L-oligonucleotide aptamers that possess outstanding binding affinities and functionality. These molecules are termed "Spiegelmer" from the German "Spiegel" or mirror. Based on the simple concept that if an aptamer binds its natural target, the mirror image of the aptamer will identically bind the mirror image of the natural target. If the process of aptamer selection is carried out against the mirror image target, an aptamer against this unnatural mirror image will be obtained. The corresponding mirror image nudeic acid (L-oligonucleotide) of this aptamer, or the Spiegelmer, should - and will - now bind to the natural target ligand with similar binding characteristics as the aptamer itself. More important, this Spiegelmer is now resistant to nuclease degradation. Spiegelmers possess the high affinity binding characteristics of the best aptamers and antibodies in the low nanomolar and picomolar range, while defying enzymatic degradation that severely limits the utility of aptamers. Data indicate that Spiegelmers are stable in human plasma for over 60 hours at 37°C, while non-modified RNA aptamers are degraded in seconds under the same conditions. Results in animals indicate that a similar stability can be expected in vivo as well. Spiegelmers should not be confused with antisense RNAs in that they do not directly interfere with protein synthesis of their target molecules. They are designed to bind specifically to extracellular molecules, either a receptor or its ligand, similar to the behavior of a monoclonal antibody, aptamer or peptide. However, recent findings demonstrate that Spiegelmers are capable of entering cells and interfering with intracellular processes as well. Finally, Spiegelmers appear to be non-immunogenic, even under the most inductive conditions for antibody formation in rabbits. This situation is of critical importance if Spiegelmer therapy is to be used repeatedly to treat chronic diseases. It is therefore preferred in the context of the present invention, to detect substances which bind or react or interact with the agonistic autoantibodies against the beta-2 receptor. These Spiegelmers can be used as peptidomimetic. It is also preferred to detect substances which block Spiegelmers that block the antigen binding site of the agonistic autoantibodies.

Also claimed by the invention are RNAi-pharmaceuticals for blocking the plasma cells which produce the agonistic autoantibodies directed against the beta-2 receptor and/or the m2 muscarinic receptor.

The invention also relates to a pharmaceutical composition comprising the inventive nudeic acid molecules, vectors, host cells, peptides and/or recognition molecules, optionally together with a pharmaceutically tolerable carrier. In particular, the pharmaceutical composition can be used as a drug. To this end, it is possible, for example, to modify the peptides by means of cyclization or other procedures well-known to those skilled in the art such that destruction thereof by endogenous peptide-degrading structures, e.g. serum proteases, is prevented. By using the peptides or recognition molecules according to the invention, *in vivo* or *ex vivo* neutralization of autoantibodies is possible. In *in vivo* neutralization, the drugs are administered directly to the patient; in *ex vivo* neutralization, the blood is conducted out of the body e.g. via a loop, e.g. in the form of a tube circulation, subsequently contacted with the drug and, following neutralization of the autoantibodies, resupplied into the organism, i.e., the patient. Regarded as drugs in the meaning of the invention are compositions for therapeutic and prophylactic purposes, as well as pharmaceutical compositions usable as diagnostic agents.

According to the invention, drugs or pharmaceutical compositions - used in a synonymous fashion herein - are substances and formulations of substances intended to cure, alleviate or avoid diseases, illness, physical defects or pathological affection by application on or in the human body. According to the invention, medical adjuvants are substances used as active ingredients in the production of drugs.

Pharmaceutical-technical adjuvants serve to suitably formulate the drug or pharmaceutical composition and, if required during the production process only, can even be removed thereafter, or they can be part of the pharmaceutical composition as pharmaceutically tolerable carriers. Examples of pharmaceutically tolerable carriers will be given below. Drug formulation or formulation of the pharmaceutical composition is optionally effected in combination with a pharmaceutically tolerable carrier and/or diluent Examples of suitable pharmaceutically tolerable carriers are well-known to those skilled in the art and comprise e.g. phosphate-buffered saline, water, emulsions such as oil/water emulsions, various types of detergents, sterile solutions, and so forth. Drugs or pharmaceutical compositions comprising such carriers can be formulated by means of well-known conventional methods. These drugs or pharmaceutical compositions can be administered to an individual at a suitable dose, e.g. in a range of from 0,01 µg to 10 g of peptides per day and patient. Doses of from 1 µg to 1 g are preferred. Preferred is administration of doses as small in number and as low as possible, preferably a single dose. Administration can be effected on various routes, e.g. oral, intramuscular, intravenous, intraperitoneal, intraosseous, rectally, vaginally, insulation, cutaneous, percutaneous, sublingual, nasal, intraarticular, intraarterial, intralymphatic, intraosseous, subcutaneous, intracutaneous, transdermal, per inhalation, pulmonal, intrapulmonal, endobronchial, intracardial, intraneural, perineural, peridural, intrathecal, intrapleural, intravitreal, parenteral, intrarectal, intragastrointestinal, intranodal, local, intradermal or on the skin or via mucosa. Administration of nudeic acids encoding the peptide according to the invention can also be effected in the form of a gene therapy, e.g. via viral vectors. The kind of dosage and route of administration can be determined by the attending physician according to clinical factors. As is familiar to those skilled in the art, the kind of dosage will depend on various factors such as size, body surface, age, sex, or general health condition of the patient, but also on the particular agent being administered, the time period and type of administration and on other medications possibly administered in parallel. Those skilled in the art will also be familiar with the fact that the concentration of autoantibodies can be diagnosed first, using the peptides according to the invention, in order to determine the required concentration of drug.

More specifically, the pharmaceutical compositions or drugs comprise a pharmacological substance which includes one or more peptides or recognition molecules of the invention, or/and nucleic acid molecules encoding same, in a suitable solution or administration form. Administration thereof can be effected either alone or together with appropriate adjuvants described in connection with drugs or pharmaceutical compositions, or in combination with one or more adjuvants, e.g. QS-21, GPI-0100 or other saponines, water-oil emulsions such as Montanide adjuvants, polylysine, polyarginine compounds, DNA compounds such as CpG, Detox, bacterial vaccines such as typhoid vaccines or BCG vaccines, salts such as calcium phosphates, and/or other suitable material enhancing the effect, preferably immunostimulatory molecules such as interleukins, e.g. IL-2, IL-12, IL-4 and/or growth factors such as GM-CSF. They are mixed with the peptides or recognition molecules of the invention according to well-known methods and administered in suitable formulations and dosages. Formulations, dosages and suitable components are well-known to those skilled in the art.

Obviously, the pharmaceutical composition or drug can also be a combination of two or more of the inventive pharmaceutical compositions or drugs, as well as a combination with other drugs, such as antibody therapies, chemotherapies or radiotherapies, suitably administered or applied at the same time or separately in time. The production of the drugs or pharmaceutical compositions proceeds according to per se known methods.

The invention also relates to a kit comprising the nudeic acid molecule, the vector, the host cell, the peptide and/or the recognition molecule, optionally together with instructions or information as to the pharmaceutical provision or the procedure of therapeutical treatment. For example, the information can be an instruction leaflet or other medium providing the user with information in which therapeutical procedure the above-mentioned substances should be used. In particular, the instruction leaflet includes detailed and/or important information about the therapeutical treatment. Obviously, the information need not necessarily be in the form of an instruction leaflet, and the information may also be imparted via the Internet.

The invention also relates to an apparatus for chromatography, which includes the peptides according to the invention. In a preferred embodiment the peptides are bound to a solid phase or an apparatus which catches from blood or plasma extracellular loop 2 peptides according to the human β2 receptor or the human m2 muscarinic receptor or antibody binding peptido mimetica fixed to nanoparticles, magnetic or paramagnetic particles or microspheres of cellulosis and other polymeric matrix molecules.

In another preferred embodiment the peptides are bound to a solid phase within the chromatography system.

In another preferred embodiment, anti-human immunoglobulin coupled columns are used for the removal of immunoglobulin from the plasma or blood of a patient suffering from Complex Regional Pain Syndrome. Because of the conclusion reached by the inventors that Complex Regional Pain Syndrome is caused by antibodies, especially by autoantibodies, the treatment of Complex Regional Pain Syndrome may also be effected by providing columns which remove antibodies or autoantibodies irrespectively from their specificity from the plasma or blood of a patient suffering from Complex Regional Pain Syndrome. In a preferred embodiment of the invention, the antibodies or autoantibodies which are not associated with Complex Regional Pain Syndrome can be reinfused to the patient.

In particular, the apparatus according to the invention (this may be an apparatus which features a specific ligand for human immunoglobulins, for example a beta-2-adrenergic receptor or the second extracellular loop of the beta-2-adrenergic receptor or the m2 muscarinic receptor or the peptides according to the invention) can be used to eliminate the autoantibodies from fluids of a patient or to neutralize the autoantibodies. This method is known to those skilled in the art under the term of immunoadsorption and/or plasma apheresis therapy. With the aid of immunoadsorption, immunoglobulins are removed from the blood or serum of a Complex Regional Pain Syndrome patient. Advantageously, this immunoadsorption treatment can be conducted in a stationary and ambulant fashion. It can be envisaged that the apparatus, particularly the so-called adsorber, is part of an extracorporeal blood circulation. To this end, blood is taken continuously or discontinuously from a patient's major vessel, particularly from an arm vein, and separated into single components, such as cellular and humoral components, using filtration or centrifugation. In particular, one essential blood component obtained in this fashion is blood plasma. Advantageously, the blood plasma can be passed through the apparatus of the invention and, following adsorption of the autoantibodies, returned into the patient, particularly through another vein of arms or legs, together with previously separated blood components, especially cellular components. It can also be envisaged that the specific ligand for the human immunoglobulin or the peptides are immobilized on a Sepharose matrix. The matrix can be placed in a container having a volume of e.g. 10 to 400 ml. Thereafter, the blood plasma of the patient can be passed over the matrix where the autoantibodies will be bound, thus allowing elimination thereof from the blood plasma. Those skilled in the art will be familiar with various ways of providing such solid phase-fixed peptides or a specific ligand for immunoglobulin, e.g. in the form of (i) regeneratable adsorption columns, (ii) double columns and (iii) disposable columns. The diverse wash and elution solutions, permitting high efficiency of treatment, can easily be determined by a person skilled in the art by using routine tests. By providing the teaching according to the invention, particularly the specific ligand for the human immunoglobulin or the peptides of the invention, various ways of employing the specific ligand for the human immunoglobulin or the peptides *in vivo, ex vivo* and *in vitro* in prophylaxis, diagnosis, therapy and aftercare of Complex Regional Pain Syndrome-induced, autoantibody-mediated diseases are disclosed to a person skilled in the art

The invention also relates to a method for the treatment of Complex Regional Pain Syndrome by binding and/or removing autoantibodies by means of peptides or specific ligands for human immunoglobulin/auto-immunoglobulin of the invention bound to a solid phase.

In a preferred embodiment of the invention the autoantibodies are directed against the human beta-2-adrenergic receptor and/or the m2 muscarinic receptor.

Also preferred is the use of the peptides of the invention in treatment and/or diagnostic of Complex Regional Pain Syndrome, wherein the peptides are administered to a patient. The peptides can be administered oral, intramuscular, intravenous, intraperitoneal, intraosseous, rectally, vaginally, insulation, cutaneous, percutaneous, sublingual, nasal, intraarticular, intraarterial, intralymphatic, intraosseous, subcutaneous, intracutaneous, transdermal, per inhalation, pulmonal, intrapulmonal, endobronchial, intracardial, intraneural, perineural, peridural, intrathecal, intrapleural, intravitreal, intrarectal, intragastrointestinal, intranodal, local, intradermal or on the skin or via mucosa and/or parenteral.

A person skilled in the art knows under which circumstances a certain kind of administration is suited most. The best kind of administration depends on several factors, like the phase of the disease, the patient's age, gender, weight and physique.

The invention also relates to the use of the nucleic acid molecules of the invention, the host cells of the invention, the vector of the invention, the peptides of the invention, the recognition molecules of the invention, the pharmaceutical composition of the invention, the kit of the invention and the apparatus of the invention in the prophylaxis, diagnosis, therapy, follow-up and/or aftercare of Complex Regional Pain Syndrome.

The invention also relates to the use of the nucleic add molecules of the invention, the host cells of the invention, the vector of the invention, the peptides of the invention, the recognition molecules of the invention, the pharmaceutical composition of the invention, the kit of the invention and the apparatus of the invention in the production of a drug for the treatment of Complex Regional Pain Syndrome.

The invention also relates to the use of a specific ligand for human immunoglobulin or the inventive peptides and nucleic acids or host cells, the pharmaceutical composition of the invention, the kit of the invention and/or the apparatus of the invention in drug screening. For example, drug screening may comprise the identification of substances, particularly peptides, proteins, carbohydrates and/or lipids, which interact with beta-2-adrenergic-receptor autoantibodies and/or m2-muscarinic-receptor-autoantibodies.

Interaction, for example, can be binding to said peptides, but also activation or inhibition of or by peptides. Accordingly, a drug can be e.g. a structure binding to the autoantibodies or - in another embodiment of the invention - to the peptide structures in the body of a patient, and consequently to the loops, thus competing with the autoantibodies for a binding site. By virtue of the disclosure of the teaching according to the invention, especially the disclosure with respect to the connection of disease and binding site of the autoantibodies, a person skilled in the art will be capable of screening various drugs. Drug screening based on disclosed targets is part of the general knowledge of a person skilled in the art and is effected using routine tests; reference is made to the corresponding standard textbooks of molecular biology and pharmacology.

The invention also relates to a method for the treatment of an autoimmune disease by binding and/or removal of autoantibodies by means of the inventive peptides bound to a solid phase. The autoantibodies are bound, complexed and/or neutralized on the solid phase by means of the peptides bound to the solid phase.

In a preferred embodiment of the invention, autoantibodies directed against the beta-2-adrenergic receptor and/or the m2 muscarinic receptor are bound, complexed and/or neutralized by means of the above-mentioned inventive materials and products, apparatus, particularly the chromatography apparatus, and peptides. For example, the peptides can be used in the detection of autoantibodies in serums of patients, using an ELISA or other immunological detection methods well-known to those skilled in the art. For detection, it can be advantageous, for example, when the autoantibodies are bound by biotinylated or otherwise coupled peptides and separated by streptavidin-coupled supports such as magnetic particles or plates. Such a method has been described in DE 102 56 897.9 which hereby is incorporated in the disclosure of the present application. More specifically, the separated autoantibodies are detected using igG subtype-specific labeled antibodies. In the event Complex Regional Pain Syndrome, the antibodies are detected using particularly IgG1, IgG2 and/or IgG3 subtype-specific labeled antibodies. Preferably antibodies directed against the m2 muscarinic receptor are detected using IgG1 and/or IgG3 subtype-specific labeled antibodies; antibodies directed against the beta-2-adrenergic receptor are detected using IgG1 and/or IgG2 subtype-specific labeled antibodies.

The invention relates also to the use of a specific ligand for human immunoglobulins in the manufacture of a column coupled to said ligand for the treatment of a patient suffering from Complex Regional Pain Syndrome, said treatment comprising passing plasma of the patient, over the column under conditions which effect the binding of said specific ligand to immunoglobulin in the patient's plasma, thereby removing a significant portion of the immunoglobulin from the patient's plasma, and reinfusing the plasma to the patient.

Additionally, the invention relates also to the treatment of a patient suffering from Complex Regional Pain Syndrome, said treatment comprising the steps of:
(a) providing a column coupled to a specific ligand for human immunoglobulin,
(b) passing plasma of the patient over the column under conditions which effect the binding of said specific ligand to immunoglobulin in the patient's plasma, thereby removing a significant portion of the immunoglobulin from the patient's plasma, and
(c) reinfusing said plasma to the patient.

In a preferred embodiment, the treatment of a patient is **characterized in that** the specific ligand is selected from the group consisting of polyclonal anti-human immunoglobulin antibodies, monoclonal anti-human immunoglobulin antibodies, a fragment of such antibodies, recombinant molecules of the antibody idiotype, synthesized peptides, Protein A and Protein G.

In another preferred embodiment of the treatment of a patient, the specific ligand recognizes antibodies directed associated with Complex Regional Pain Syndrome. Preferably, the specific ligand is an antigen-mimicking molecule selected from the group consisting of polyclonal and monoclonal antiidiotypic antibodies, fragments of such antibodies, and synthesized peptides.

Preferably, the specific ligand is a synthesized peptide mimicking a sequence of a receptor structure.

In another preferred embodiment of the treatment of a patient, the receptor is the beta-2-adrenergic receptor and/or the m2 muscarinic receptor.

Preferably, the autoantibodies are directed against a molecule selected from the group consisting of beta-2-adrenergic receptors and/or the m2 muscarinic receptor.

In another preferred embodiment of the treatment of a patient, the invention is characterized by a parallel or subsequent combination with β-blockers and/or m2 muscarinic receptor blockers or intravenous immunoglobulin.

In the context of the invention is a treatment of a patient suffering from Complex Regional Pain Syndrome a preferred embodiment of the use of the specific ligand for human immunoglobulin in the manufacture of a column for the treatment of a patient.

Additionally, the invention relates also to the treatment of a patient suffering from Complex Regional Pain Syndrome, said treatment comprising the administration of the peptides of the invention into the patients body. Also preferred is the administration of a mixture comprising more than one peptide of the invention.
It was very surprising that Complex Regional Pain Syndrome can be treated by injecting the peptides of the invention, without causing any side effect.
This is a preferred method for the treatment, because it is less stressing and more convenient for the patient.
The use of cyclic peptides comprising the peptides of the invention is especially preferred due to their resistance to digestion. Therefore cyclic peptides are advantageous for example for oral use. Surprisingly the cyclic peptides of the invention which were injected into a patent's body also showed superior results. The general condition of the patients treated with cyclic peptides was improved considerably.

Also preferred is the use of the peptides of the invention in parallel or subsequent combination with intravenous immunoglobulin and/or corticosteroides. Surprisingly these combinations resulted in an especially fast improvement of the general conditions of the patients.

The teachings of the present invention are characterised by the following features:
- departure from the beaten track
- a new perception of the problem
- satisfaction of a long-felt need or want
- hitherto all efforts of experts were in vain
- the simplicity of the solution, which proves inventive action, especially since it replaces a more complex doctrine
- the development of scientific technology followed another direction
- the achievement forwards the development
- misconceptions among experts about the solution of the according problem (prejudice)
- technical progress, such as: improvement, increased performance, price-reduction, saving of time, material, work steps, costs or resources that are difficult to obtain, improved reliability, remedy of defects, improved quality, no maintenance, increased efficiency, better yield, augmentation of technical possibilities, provision of another product, opening of a second way, opening of a new field, first solution for a task, spare product, alternatives, possibility of rationalisation, automation or miniaturisation or enrichment of the pharmaceutical fund
- spedal choice; since a certain possibility, the result of which was unforeseeable, was chosen among a great number of possibilities, it is a patentable lucky choice
- error in citations
- young field of technology
- combined invention; a combination of a number of known elements, with a surprising effect
- licensing
- praise of experts and
- commercial success

Said advantages are shown especially in the preferential embodiments of the invention.

Without intending to be limiting, the invention will be explained in more detail with reference to the examples.

### Examples

### Example 1:

### IgG autoantibodies from CRPS patients show agonistic effects on the beta2-adrenoceptor and the muscarinic m2 receptor

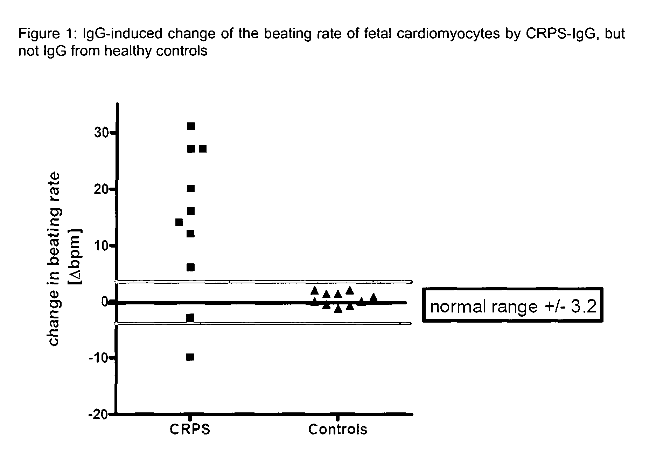

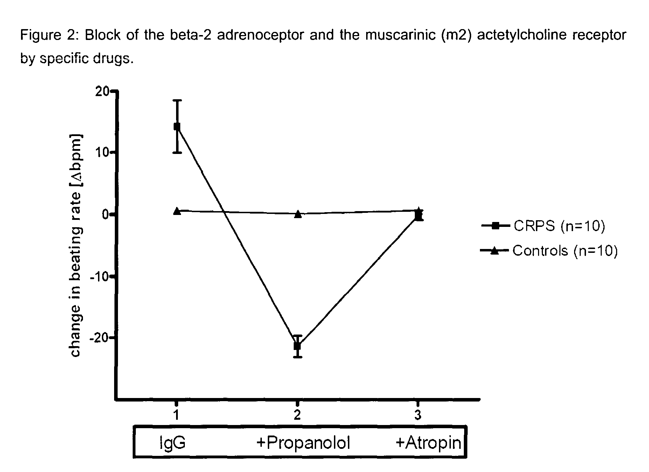

Incubation of spontaneous beating rat fetal cardiomyocytes with isolated IgG from CRPS patients induced an increase of the beating rate in 80% of the CRPS , but none of the control IgG (p< 0.01, Fig.1). This effect was then blocked with the beta-blocker propanolol in a concentration with no intrinsic effect. The beating rate of the CRPS-IgG-treated cardiomyocytes decreased below the basic level, whereas cardiomyocytes incubated with control IgG showed no effect (Fig.2). Blocking with atropine increased the beating rate of the CRPS-IgG treated cardiomyocytes to basic level and again the controls remained unaffected. Therefore an additional agonistic effect on the muscarinic m2 acetlycholine receptor was suspected.

### Example 2:

### IgG autoantibodies of CRPS patients bind to epitopes on the second extracellular loop of the beta2-and/or m2 receptor

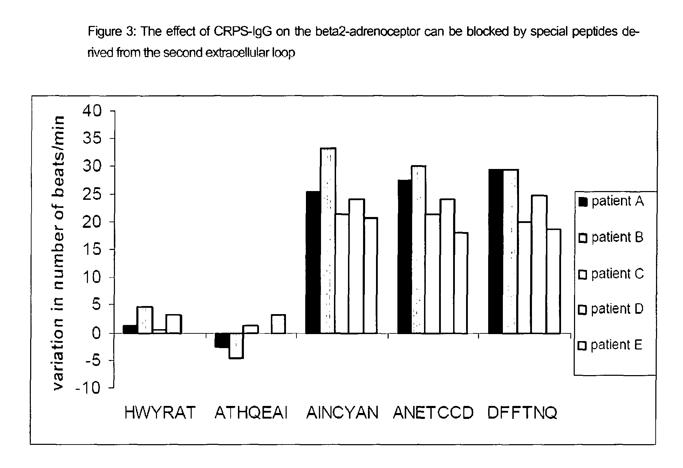

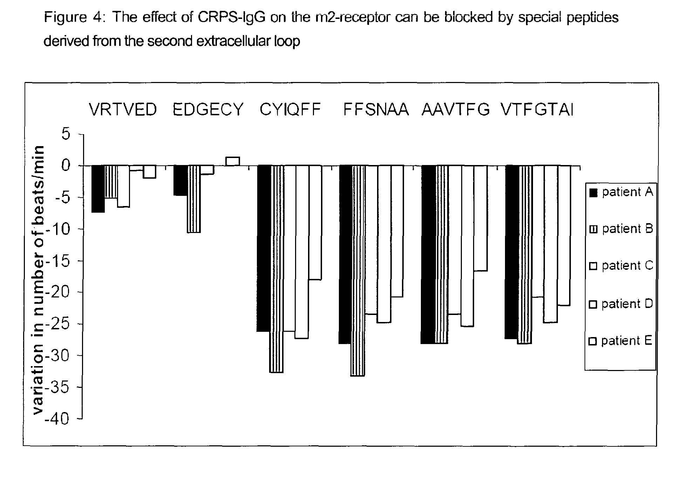

CRPS IgG was incubated with peptides according to the sequences of parts of the 2^{nd} extracellular loop of the beta2 adrenoceptor (Table 1) and then incubated with fetal cardiomyocytes.
Two out of the five peptides used were able to inhibit the aforementioned increase in beating rate (Fig.3).
The same experiment was done for peptides derived from the sequence of the 2^{nd} extracellular loop of the muscarinic m2 receptor (Table 1). The cardiomyocytes were preincubated with propanolol to prevent the beta2-agonistic effect. Then the CRPS-lgG was incubated with the peptides and then given to the cardiomyocytes. Two out of five peptides blocked the negative chronotrophic effect nearly complete (Fig. 4)

### Example 3:

### Establishing a test system for autoantibodies in CRPS using receptor-transfected cell lines

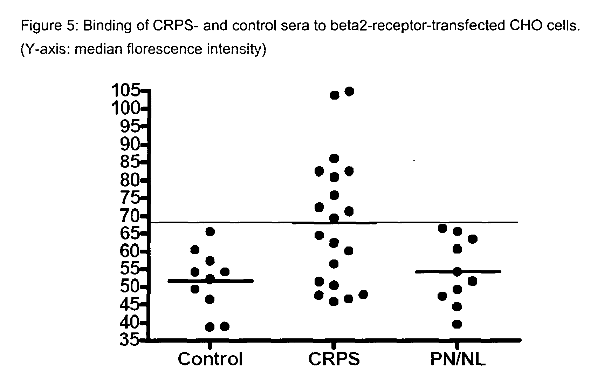

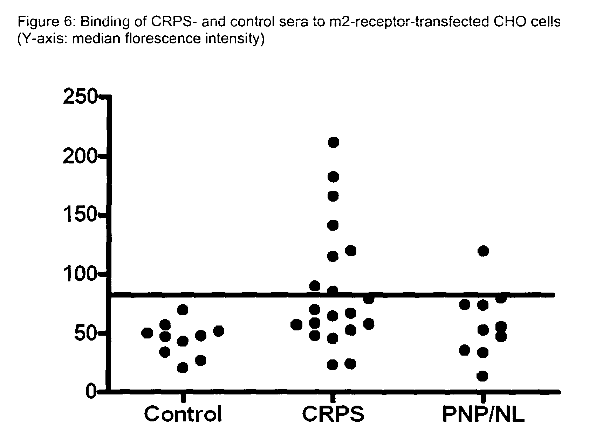

After transfection of beta2 adrenoceptors in chinese hamster ovary (CHO) cells, binding to transfected, but not to untransfected cells was detectable in 50% of the CRPS patients. None of the control patients showed binding to the beta2-transfected cells (Fig. 5). CHO cells transfected with m2 receptor showed binding in 40% of the CRPS patients, but not in healthy controls and only one patient with a nerve lesion was positive (Fig. 6).

### Example 4:

### Subclass specificity of the receptor-binding autoantibodies

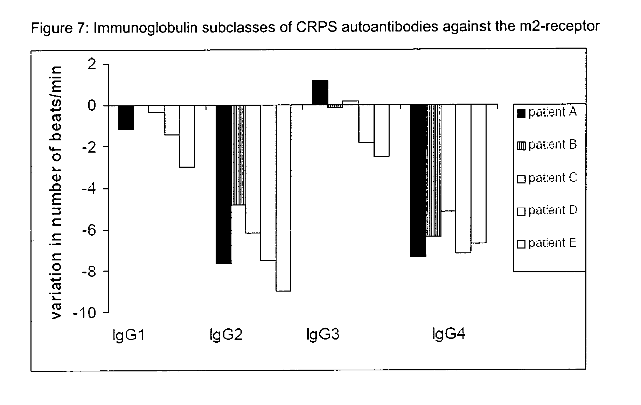

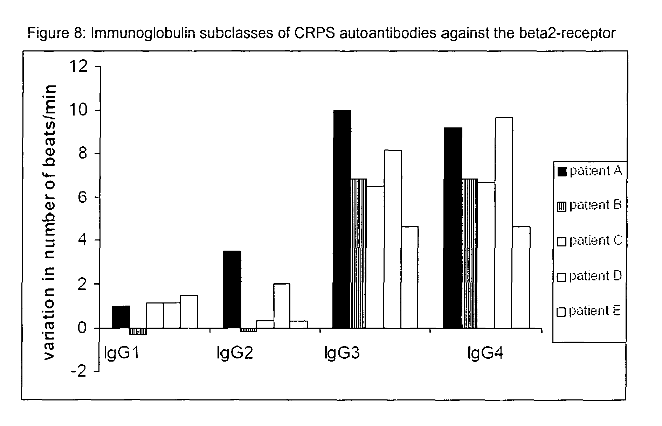

After preincubation of the CRPS IgG with subclass-specfic anti-human IgG antibodies, the beta2-effect could be blocked by anti-IgG1 and anti-IgG2, whereas the m2-receptor effect could be blocked by anti-IgG1 and anti-IgG3 (Fig.7 and 8)

**Table 1: Peptide sequences derived from 2^{nd} extracellular loop of the beta2 adrenoceptor and the muscarinic m2 receptor**

| Beta2 adrenoceptor | Muscarinic m2 acetylcholine receptor |
|---|---|
| HWYRAT | VRTVED |
| ATHOEAI | EDGECY |
| AINCYAN (SEQ ID NO 9) | CYIQFF (SEQ ID NO 12) |
| ANETCCD (SEQ ID NO 10) | FFSNAA (SEQ ID NO 13) |
| DFFTNQ (SEQ ID NO 11) | AAVTFG (SEQ ID NO 14) |
| | VTFGAI (SEQ ID NO 15) |

### Conclusion

1. Binding studies and functional studies show, that only CRPS patients, but not controls bind to and affect the function of the beta2 adrenoceptor and / or the muscarinic m2 acetylcholine receptor, which allows the diagnosis of CRPS for the first time using a biochemical diagnostic assay.
2. The ability of small peptides to block the functional effects of these autoantibodies suggests, that they can be used for the treatment of the CRPS.
3. The results suggest, that autoantibodies against the beta2 adrenoceptor lead to an increased beating rate, whereas autoantibodies against the muscarinic m2 acetylcholine receptor cause a decreased beating rate. Both phenomena play a role in the etiology of Complex Regional Pain Syndrome.

### Example 5:

### ELISA

NUNC microtiter plates (Nunc, Kastrup, Denmark) were coated with 50 ug/mL of synthetic peptides corresponding to the sequences of the second extracellular loop of both the human β2 adrenergic receptor and muscarinic 2 receptor in 100mM carbonate-bicarbonate buffer (Na2CO3, pH 9.6) overnight at 4°C. In order to avoid unspecific binding, blocking solution (phosphate buffered saline + 0.1 % Tween 20 - PBST - supplemented with 5% BSA) was added to the wells and the plates maintained at room temperature for 1 h. After three washings, affinity-purified 30uM of immunoglobulins from CRPS patients and healthy controls were allowed to adsorb on the peptides for 2 hour at room temperature. After washing the wells three times with PBST, bound antibodies were revealed by successive 2 h incubations at room temperature with a peroxidase conjugated anti-mouse IgG (diluted 1:6000 in PBST). This was again followed by three washings and then a TMB Peroxidase substrate (KPL, Gaithersburg, USA) was added. After 10 min, 100ul of 1 N HCl were added to stop the reaction. The optical density was measured at 450nm in an ELISA plate reader.

## Claims

1. A peptide, consisting of an amino acid sequence X1-X2-X3-X4-X5-X6-X7-X8-X9-X10-X11-X12-X13 or parts thereof, wherein
| | |
|---|---|
| X1 = | amino group, amide, acetyl group, biotin group, marker, spacer, linker, GKK, SGKK or deletion, |
| X2 = | H, A, V, E, L, D, I, N |
| X3 = | W, T, R, D, F, Y, S, C, K, G, E |
| X4 = | Y, H, T, G, S, C, R, K |
| X5 = | R, Q, V, E, K, G, N, D, I, L, A |
| X6 = | A, E, D, C, L, V, Y, S, T, N |
| X7 = | T, A, Y, D, G, S, C, L, V, K, R, Q |
| X8= | I,H,E,G,L,V,D,N,K |
| X9 = | Q, C, E, N, S, T |
| X10= | E, Y, C, D, S, T, N, M |
| X11= | A, Y, L, V, S, T |
| X12= | I, L, V |
| X13= | amino group, amide, acetyl group, biotin group, marker, spacer, linker, GKK, SGKK or deletion; |
wherein the amino acid sequence is not VRTVED, EDGECY, or VRTVEDGECY.

2. The peptide according of claims 1,
**characterized in that**
it consists of the amino acid sequence HWYRAT, ATHQEAI and/or HWYRATHQEAI.

3. A peptide of claims 1 or 2 for use as a medical active substance.

4. A peptide consisting of an amino acid sequence X1-X2-X3-X4-X5-X6-X7-X8-X9-X10-X11-X12-X13 or parts thereof, wherein
| | |
|---|---|
| X1 = | amino group, amide, acetyl group, biotin group, marker, spacer, linker, GKK, SGKK or deletion, |
| X2 = | H, A, V, E, L, D, I, N |
| X3 = | W, T, R, D, F, Y, S, C, K, G, E |
| X4 = | Y, H, T, G, S, C, R, K |
| X5 = | R, Q, V, E, K, G, N, D, I, L, A |
| X6 = | A, E, D, C, L, V, Y, S, T, N |
| X7 = | T, A, Y, D, G, S, C, L, V, K, R, Q |
| X8 = | I, H, E, G, L, V, D, N, K |
| X9 = | Q, C, E, N, S, T |
| X10= | E, Y, C, D, S, T, N, M |
| X11= | A, Y, L, V, S, T |
| X12= | I, L, V |
| X13= | amino group, amide, acetyl group, biotin group, marker, spacer, linker, GKK, SGKK or deletion; |
for use as a binding peptide to antibodies associated with Complex Regional Pain Syndrome.

5. The peptide according to the preceding claim,
**characterized in that**
it consists of the amino acid sequence HWYRAT, ATHQEAI, HWYRATHQEAI, VRTVED, EDGECY, and/or VRTVEDGECY.

6. The peptide according to any of claims 1 to 5 in a linear and branched as well as cyclic form, wherein the peptide ring closure is effected through disulfide bridging when two cysteines are present, or through amide cyclization, which is optionally effected through side chains, through the C and N termini or through a combination of these three possibilities.

7. The peptide according to any of claims 1 to 6, **characterized in that**
said peptide binds immunoglobulins or antigen-immunoglobulin complexes in biological fluids.

8. The peptides according to any of claims 1 to 7,
**characterized in that**
the immunoglobulins are agonistic autoantibodies which interact with the beta-2-adrenergic receptor and/or the m2 muscarinic receptor.

9. Use of the beta-2-adrenergic receptor and/or the m2 muscarinic receptor and/or parts thereof, preferably the peptides according to claims 1 to 8 for the production of agents for diagnostic and/or therapeutic purposes in Complex Regional Pain Syndrome.

10. Use of at least one specific ligand for human immunoglobulin in the manufacture of a column coupled to said ligand for the treatment of a patient suffering from Complex Regional Pain Syndrome, said treatment comprising passing plasma of the patient, over the column under conditions which effect the binding of said specific ligand to immunoglobulin in the patient's plasma, thereby removing a significant portion of the immunoglobulin from the patient's plasma, and reinfusing the plasma to the patient.

11. Use of at least one specific ligand for human immunoglobulin in the manufacture of a column coupled to said ligand for the diagnosis of a patient, said treatment comprising passing plasma of the patient, over the column under conditions which effect the binding of said specific ligand to immunoglobulin in the patient's plasma, if present, thereby removing a significant portion of the immunoglobulin from the patient's plasma, and reinfusing the plasma to the patient.

12. Use of the peptide of any of the claims 1 to 6 in treatment and/or diagnostic of Complex Regional Pain Syndrome, wherein the peptide is administered to a patient preferred orally, intramuscular, intravenous, intraperitoneal, intraosseous, rectally, vaginally, insufflation, cutaneous, percutaneous, sublingual, nasal, intraarticular, intraarterial, intralymphatic, intraosseous, subcutaneous, intracutaneous, transdermal, per inhalation, pulmonal, intrapulmonal, endobronchial, intracardial, intraneural, perineural, peridural, intrathecal, intrapleural, intravitreal and/or parenteral. intrarectal, intragastrointestinal, intranodal, local, intradermal or on the skin or via mucosa.

13. Use of a specific ligand for human immunoglobulin in the manufacture the device for an Assay preferred an ELISA for the diagnosis of Complex Regional Pain Syndrome.

14. A method for removing a portion of the immunoglobulin from plasma taken from a patient suffering from Complex Regional Pain Syndrome, the method comprising
(a) providing a column coupled to a specific ligand for human immunoglobulin, which column is as defined in any one of claims 10 or 11; and
(b) passing the plasma over the column under conditions which effect the binding of said specific ligand to immunoglobulin in the plasma.

15. An isolated nucleic acid molecule selected from the group comprising:
a) a nucleic acid molecule comprising a nucleotide sequence which encodes at least one peptide selected from the group consisting of peptides according to claim 1;
b) a nucleic acid molecule which is complementary to a nucleotide sequence in accordance with a);
c) a nucleic acid molecule which undergoes hybridization with a nucleotide sequence according to a) or b) under stringent conditions;
d) a nucleic add molecule comprising a nucleotide sequence having sufficient homology to be functionally analogous to a nucleotide sequence according to a), b) or c);
e) a nucleic acid molecule which, as a consequence of the genetic code, is degenerated into a nucleotide sequence according to a) through d); and
f) a nucleic acid molecule according to a nucleotide sequence of a) through e) which is modified by deletions, additions, substitutions, translocations, inversions and/or insertions and functionally analogous to a nucleotide sequence according to a) through e)

16. The peptide according to any of the claims 1 to 8,
**characterized in that**
the linker and/or a spacer are selected from the group comprising:
α-aminocarboxylic acids as well as homo- and heterooligomers thereof, α,ω-aminocarboxylic acids and branched homo- or heterooligomers thereof, other aliphatic and/or aromatic amino adds as well as linear and branched homo- or heterooligomers; amino-oligoalkoxylamines; maleinimidocarboxylic acid derivatives; oligomers of alkylamines; 4-alkylphenyl derivatives; 4-oligoalkoxyphenyl or 4-oligoalkoxyphenoxy derivatives; 4-oligoalkylmercaptophenyl or 4-oligoalkylmercaptophenoxy derivatives; 4-oligo-alkylaminophenyl or 4-oligoalkylaminophenoxy derivatives; (oligoalkylbenzyl)phenyl or 4-(oligoalkylbenzyl)phenoxy derivatives, as well as 4-(oligo-alkoxybenzyl)phenyl or 4-(oligoalkoxy-benzyl)phenoxy derivatives; trityl derivatives; benzyloxyaryl or benzyloxyalkyl derivatives; xanthen-3-yloxyalkyl derivatives; (4-alkylphenyl or ω-(4-alkylphenoxy)alkanoic acid derivatives; oligoalkylphenoxyalkyl or oligoalkoxyphenoxyalkyl derivatives; carbamate derivatives; amines; trialkylsilyl or dialkylalkoxysilyl derivatives; alkyl or aryl derivatives and/or combinations thereof.

17. The peptide according to any of the claims 1 to 8 or 16,
**characterized in that**
it is selected from the group consisting of:
a) a peptide consisting of the amino acid sequence X1-X2-X3-X4-X5-X6-X7-X8-X9 - X10-X11-X12 -X13 or parts thereof according to claim 1;
b) a peptide consisting of an amino acid sequence having sufficient homology to be functionally analogous to an amino add sequence in accordance with a);
c) a peptide according to an amino acid sequence a) or b) which is modified by deletions, additions, substitutions, translocations, inversions and/or insertions and functionally analogous to an amino acid sequence in accordance with a) or b);
d) a peptide according to amino acid sequence a), b) or c) which is modified by branch or extension with the same or another peptide according to amino add sequence a), b) or c) to form a homooligomeric or heterooligomeric peptide.

18. The peptide according to claim 17,
**characterized in that**
the amino acid sequence specified under b) has at least 40% homology to any of the amino acid sequences specified under a).

19. The peptide according to claim 17 or 18,
**characterized in that**
the amino acid sequence specified under b) has at least 60%, preferably 70%, more preferably 80%, especially preferably 90% homology to any of the amino acid sequences specified under a).

20. The peptide according to any of claims 1 to 8,16 to 19,
**characterized in that**
the peptide is immobilized and/or fixed to magnetic, paramagnetic and/or non magnetic nanoparticles.

21. The peptide according to the preceding claim,
**characterized in that**
the peptide is bound to a solid phase.

22. The peptide according to any of claims 1 to 8,16 to 21,
**characterized in that**
it additionally comprises amino groups, amides, acetyl groups, biotin groups, markers, spacers and/or linkers.

23. The nucleic acid molecule according to claim 15,
**characterized in that**
the nucleotide sequence specified under d) has at least 40% homology to any of the nucleotide sequences specified under a) through c).

24. The nucleic acid molecule according to claim 15,
**characterized in that**
the nucleotide sequence specified under d) has at least 60%, preferably 70%, more preferably 80%, especially preferably 90% homology to any of the nucleotide sequences specified under a) through c).

25. The nucleic acid molecule according to any of claims 15, 23 or 24,
**characterized in that**
it is a genomic DNA, a cDNA and/or an RNA.

26. A vector comprising a nucleic acid molecule according to any of claims 15, 23 to 25.

27. A host cell comprising the vector according to claim 25, a nucleic acid molecule according to any of claims 15 or 23 to 25 and/or a peptide according to any of claims 1 to 3.

28. A peptide, said peptide being encoded by a nucleic acid molecule according to any of claims 15 or 23 to 25.

29. Solid phases for affinity chromatography or solid-phase extraction consisting of organic, inorganic, synthetic polymers or of mixed polymers, preferably cross-linked agarose, cellulose, silica gel, polyamide and polyvinyl alcohols, which are optionally chemically activated, with peptides according to at least one of claims 1 to 8 or 16 to 22, immobilized on the surface of the solid phase.

30. The solid phases according to claim 29, wherein the peptides are bound to the solid support phase covalently or by adsorption.

31. The solid phases according to claim 29 or 30, wherein the peptides are covalently bound to the solid phase on position X1, X2, X3, X4, X5, X6, X7, X8, X9, X10, X11, X12 and/or X13.

32. The solid phases according to any of claims 29 to 31, wherein the peptides are distanced from the support surface by linkers/spacers.

33. A device for removing immunoglobulins from immunoglobulin-containing samples on solid phases, wherein the device contains a solid phase according to any of claims 29 to 32, and means for the entry of immunoglobulin-containing samples are provided.

34. A pharmaceutical composition comprising a nucleic acid molecule according to any of claims 15 or 23 to 25, a vector according to claim 26, a host cell according to claim 27, a peptide according to any of claims 1 to 8 or 17 to 22, and/or a solid phase according to claims 29 to 32, optionally together with a pharmaceutically tolerable carrier.

35. A kit comprising a nucleic acid molecule according to any of claims 15 or 23 to 25, a vector according to claim 26, a host cell according to claim 27, a peptide according to any of claims 1 to 8 or 17 to 22, a solid phase according to claims 29 to 32 and/or a pharmaceutical composition according to claim 34, optionally together with instructions for combining the contents of the kit and/or providing a formulation.

36. An apparatus for chromatography, comprising peptides according to any of claims 1 to 8 or 17 to 22.

37. The apparatus according to claim 36,
**characterized in that**
the peptides are bound to a solid phase according to claims 29 to 32.

38. Use of a nucleic acid molecule according to any of claims 15 or 23 to 25, a vector according to claim 26, a host cell according to claim 27, a peptide according to any of claims 1 to 8 or 17 to 22, a solid phase according to claims 29 to 32, a pharmaceutical composition according to claim 34, a kit according to claim 35, an apparatus according to claim 36 or 37 in the prophylaxis, diagnosis, therapy, follow-up and/or aftercare of Complex Regional Pain Syndrome.

39. Use of a nucleic acid molecule according to any of claims 15 or 23 to 25, a vector according to claim 26, a host cell according to claim 27, a peptide according to any of claims 1 to 8 or 17 to 22, a solid phase according to claims 29 to 32, a pharmaceutical composition according to claim 34, a kit according to claim 35, an apparatus according to claim 36 or 37 in the production or screening of a drug for the treatment of Complex Regional Pain Syndrome.

40. The use according to claim 9, 38 and/or 39,
**characterized in that**
the Complex Regional Pain Syndrome is a Reflex Sympathetic Dystrophy (RSD), a Causalgia, a Sudeck's atrophy, an algodystrophy, a neurodystrophic Syndrome, a dystrophic Pseudoarthritis, Reflex Neurovascular Dystrophy (RND), an Algoneurodystrophy and/or a transiente Osteoporose.

41. The use according to any of claims 9, 38 to 40,
**characterized in that**
a peptide according to any of claims 1 to 8 or 17 to 22 is used to detect, bind, complex and/or neutralize autoantibodies directed against beta-2-adrenergic receptor and/or m2 muscarinic receptor.

42. The use according to claim 41,
**characterized in that**
the antibodies are IgG1, IgG2 and/or IgG3 subtype-specific antibodies.

43. A method for the treatment, prophylaxis, diagnosis, follow-up and/or aftercare of Complex Regional Pain Syndrome by binding and/or removal of autoantibodies by means of peptides according to any of claims 1 to 8 or 17 to 22 bound to a solid phase.

44. The method according to claim 43,
**characterized in that**
the autoantibodies are directed against the second extracellular loop of the human β2 adrenergic receptor and/or its polymorphisms.

45. The method according to claim 43,
**characterized in that**
the autoantibodies are directed against the second extracellular loop of the human m2 muscarinic receptor and/or its polymorphisms.

46. Use of a specific ligand according to claim 10 or 11 wherein selective IgG3 apheresis from blood or plasma to treat Complex Regional Pain Syndrome is used.

47. Use according to any of the claims 10, 11 or 46, wherein said specific ligand is selected from the group consisting of polyclonal anti-human immunoglobulin antibodies, monoclonal anti-human immunoglobulin antibodies, a fragment of such antibodies, recombinant molecules of the antibody idiotype, synthesized peptides, Protein A and Protein G and Plasmapheresis and removal of autoantibodies which react with the beta-2 receptor and/or the m2 muscarinic receptor or all IgG from whole blood.

48. Use according to claim 10, 11, 46 or 47, wherein said specific ligand is an antigen-mimicking molecule selected from the group consisting of polyclonal and monoclonal antiidiotypic antibodies, fragments of such antibodies, and synthesized peptides.

49. Use according to claim 10, 11, 46 to 48 wherein said specific ligand is an antigen-mimicking molecule of HWYRAT, ATHQEAI, HWYRATHQEAI, VRTVED, EDGECY, and/or VRTVEDGECY, selected from the group consisting of polyclonal and monoclonal antiidiotypic antibodies, fragments of such antibodies, and synthesized peptides.

50. Use according to claim 10, 11, 46 to 49, wherein said specific ligand is a synthesized peptide mimicking a sequence of a receptor structure.

51. Use according to claim 10, 11, 46 to 50, wherein said receptor is the beta-2-adrenergic receptor and/or m2 muscarinic receptor.

52. Use according to claim 10, 11, 46 to 51 wherein said autoantibodies are directed against a molecule selected from the group consisting of beta-2-adrenergic receptors and/or m2 muscarinic receptor.

53. Use according to claim 10, 11, 46 to 52 in parallel or subsequent combination with intravenous immunoglobulin and/or corticosteroides.
